# EUROPEAN PATENT APPLICATION

(11) **EP 3 818 961 A1**
(43) Date of publication of application: **12.05.2021**
(21) Application number: 20175102.1
(22) Date of filing: 15.05.2020
(51) Int. Cl.: A61F 2/06, A61L 27/18, A61L 27/34, A61L 27/50, A61L 27/56

(54) **POLYMER FIBER TUBULAR STRUCTURE AND PREPARATION METHOD THEREOF**

(30) Priority: 08.11.2019 TW 108140542; 08.11.2019 TW 108140543
(71) Applicant: Coating P. Materials Co., Ltd., Taichung 408 (TW)
(72) Inventor: Chen, Shih-Hsien, 804 Kaohsiung City (TW); Chang, Cin-He, 403 Taichung City (TW); Lin, Yung-Tai, 407 Taichung City (TW)
(74) Representative: Lang, Christian

(57) **Abstract**

A polymer fiber tubular structure includes a first pipe element, a second pipe element, and a coil winding structure, wherein the first pipe element includes an inner surface and an outer surface and is composed of silicon-containing polycarbonate type polyurethane elastomer, the second pipe element includes an inner surface and an outer surface and is composed of polycarbonate type polyurethane elastomer. The second pipe element is wrapped on the outer surface of the first pipe element such that the first pipe element and the second pipe element are concentric structures. The coil winding structure is provided for embedding into the outer surface of the first pipe element or into the outer surface of the second pipe element, thereby, the kinking of the polymer fiber tubular structure is to be reduced during use, and the thrombosis can be further avoided when the polymer fiber tubular is used for the human being.

## Description

### CROSS-REFERENCE TO RELATED APPLICATIONS

This application claims the benefit of TW 108140542, filed on November 08, 2019 and TW 108140543, filed on November 08, 2019, the content of which are hereby incorporated by reference in their entirety.

### FIELD OF THE INVENTION

The present invention provides an artificial blood vessel, and more particularly, a polymer fiber tubular structure that can prevent kinking of pipes in artificial blood vessel.

### BACKGROUND OF THE INVENTION

According to the 2014 World Health Organization (WHO) report, in recent years, due to the factors of continued aging of the global population, excessive urbanization, and unhealthy lifestyles, a striking high proportion of four-out-of-five (82%) deaths, contributed by four major noncontagious diseases, cancer, diabetes, cardiovascular disease and chronic respiratory diseases. It is worth noting that the number of patients with cardiovascular disease and diabetes has been increasing year by year. Therefore, the demand for artificial blood vessels has also increased globally due to the trend. As of 2015, the global demand for artificial blood vessels has reached the number of 290,000 pieces.

At present, the surgical treatment for vascular occlusion in lower limbs is to perform bypass procedure with artificial blood vessels, but since the current existing artificial blood vessel products contain very different properties from human blood vessels (such as mechanical properties, biocompatibility, etc.), when applied to the blood vessels with smaller diameter, such as the peripheral blood vessels of the lower limbs, patients often have poor post-surgical outcome; other treatment methods, such as: balloon dilatation, are not very effective, and the recurrence rate of vascular occlusion within three months after surgery is still calculated up to 70%.

Polyurethane (PU) is a polymer material with great adjustable mechanical properties, and also contains good biocompatibility and material stability. Currently, many studies have introduced this material to the small blood vessel bypass surgery around the lower limbs, and have obtained good results in animal experiments. The data shows that polyurethane has considerable potential for the small blood vessel bypass treatment around the lower limbs.

Clinically, when thrombus or stenosis formation occurs in blood vessels, and when evaluations suggest the best treatment option is surgery, the patient's own veins are the first choice for transplantation, if no suitable veins are available for use, the use of artificial blood vessels made of synthetic materials are taken into consideration as a substitute for vascular transplantation. However, currently the main materials of commercially available artificial blood vessels for bypass are ePTFE and Dacron. These two materials when used in low-resistance large-diameter artificial blood vessels (> 6 mm), shows good dimensional stability and patency, but when used in high-resistance small-diameter artificial blood vessels, due to the huge differences in compliance, elasticity, and flexibility between the material and the human blood vessels, the shear generated at the junction of blood vessels (shear stress), formation of turbulent flow, and intimal hyperplasia lead to thrombosis, which further results in poor post-surgical outcomes.

Another problem caused by using of artificial blood vessels is the formation of kinks. For example, when an artificial blood vessel is implanted in a patient's body, due to bending the artificial blood vessel often encounters the phenomenon to kink. Therefore, kinking in the implanted artificial blood vessel causes blockage to the blood flow and result in thrombosis, which further causes poor post-surgical outcomes.

### SUMMARY OF THE INVENTION

According to the drawbacks of the prior art, the main object of the present invention is to provide a polymer fiber tubular structure that prevents tubular from kinking, and reduces the problem of thrombosis caused by blood flow obstruction.

Accordingly, the present invention provides a polymer fiber tubular structure which includes a first pipe element, a second pipe element and a coil winding structure, in which the first pipe element includes an inner surface and an outer surface and is composed of a silicon-containing polycarbonate type polyurethane elastomer. The second pipe element includes an inner and an outer surface and is composed of polycarbonate type polyurethane elastomer and the second pipe encapsulated on the outer surface of the first pipe element so that the first pipe element and the second pipe element are concentric structure. The coil winding structure is provided for embedding into the outer surface of the first pipe element or into the outer surface of the second pipe element.

According to the above problems, the present invention also provides a method for preparing a polymer fiber tubular structure, which includes forming a first pipe element with an inner surface and an outer surface and forming a second pipe element with an inner surface and an outer surface to encapsulate the outer surface of the first pipe element, so that the first pipe element and the second pipe element are concentric circles, and a coil winding structure is disposed on the outer surface of the first pipe element or on the outer surface of the second pipe element. The steps of forming the first pipe element further includes: providing a silicon-containing polycarbonate type polyurethane solution, spraying the silicon-containing polycarbonate type polyurethane solution to form a silicon-containing polycarbonate type polyurethane fiber, and collecting the silicon-containing polycarbonate type polyurethane fiber to obtain a silicon-containing polycarbonate type polyurethane elastomer to compose the first pipe element. The steps of forming the second pipe element further include: providing a polycarbonate type polyurethane solution and encapsulating the outer surface of the first pipe element by spraying the polycarbonate type polyurethane solution to form a polycarbonate type polyurethane elastomer, in which the polycarbonate type polyurethane elastomer is the second pipe element.

### BRIEF DESCRIPTION OF THE DRAWINGS

Fig. 1 is a schematic cross-sectional view of showing a polymer fiber tubular structure in accordance with the present invention disclosed herein.
Fig. 2 is a flowchart showing steps of a method for preparing a polymer fiber tubular structure in accordance with the present invention disclosed herein.
Fig. 3a is a schematic cross-sectional view showing a coil winding structure embedded into the outer surface of a first pipe element in a polymer fiber tubular structure in accordance with the present invention disclosed herein.
Fig. 3b is a schematic cross-sectional view showing a coil winding structure embedded into the outer surface of a first pipe element in a polymer fiber tubular structure in accordance with the present invention disclosed herein.
Fig. 3c is a SEM image showing the fiber structure of the inner layer of the first pipe element in the polymer fiber tubular structure in accordance with the present invention disclosed herein.
Fig. 4a is a schematic diagram showing a coil winding structure embedded into the outer surface of the second pipe element in the polymer fiber tubular structure in accordance with the present invention disclosed herein.
Fig. 4b is a schematic cross-sectional view showing a coil winding structure embedded into the outer surface of the second pipe element in the polymer fiber tubular structure in accordance with the present invention disclosed herein.
Fig. 4c is a SEM drawing showing the outer surface fiber structure of the second pipe element in the polymer fiber tubular structure in accordance with the present invention disclosed herein.
Fig. 5 is a schematic cross-sectional view of showing a coil winding structure embedded into an inner surface of the first pipe element and an outer surface of the second pipe element in a polymer fiber tubular structure in accordance with the present invention disclosed herein.
Fig. 6 is a schematic cross-sectional view of showing an adhesive layer disposed between the inner surface of the first pipe element and the second pipe element of a polymer fiber tubular structure in accordance with the present invention disclosed herein.
Fig. 7 is a schematic diagram showing SEM/EDS mapping of a double-layer fiber structure of a polymer fiber tubular structure in accordance with the present invention disclosed herein.

### DETAILED DESCRIPTION OF THE PREFERRED EMBODIMENT

In order to allow committee review members and those skilled in the art to fully understand the effects of the present invention, the drawings and figures are used to further illustrate the present invention using a preferred embodiment.

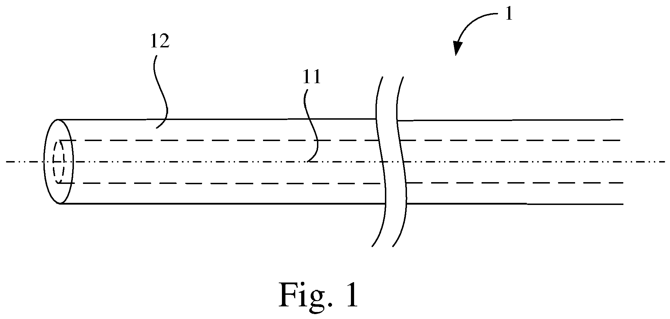

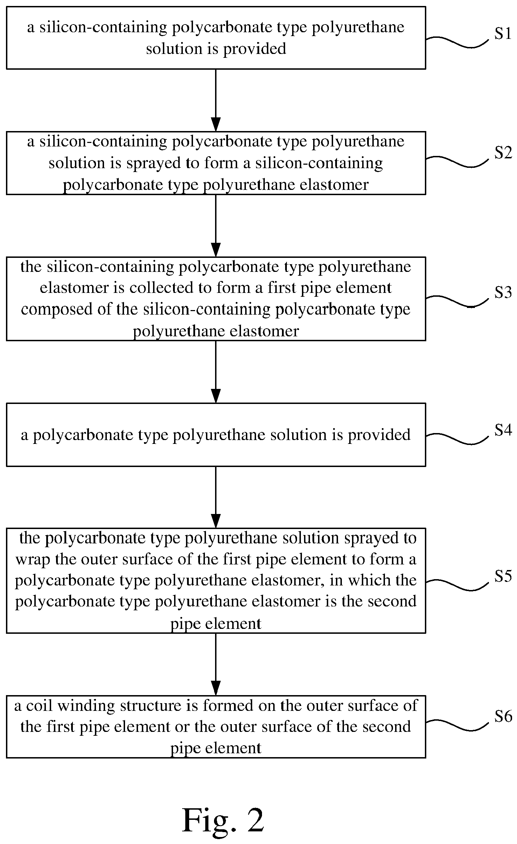

First, please refer to Fig. 1. Fig. 1 is a schematic cross-sectional view showing a double-layer structure of a polymer fiber tubular structure 1. In Fig. 1, the polymer fiber tubular structure 1 is composed of a first pipe element 11 and a second pipe element 12, in which the first pipe element 11 includes an inner surface and an outer surface and the second pipe element 12 also includes an inner surface and an outer surface, and the second pipe element 12 is wrapped the outer surface of the first pipe element 11, such that the first pipe element 11 and the second pipe element 12 are concentric structures. In the embodiment of the present invention, the material of the first pipe element 11 is a silicon-containing polycarbonate type polyurethane elastomer, and the material of the second pipe element 12 is a polycarbonate type polyurethane elastomer, and the preparing method is described in steps in the flowchart of Fig. 2.

As shown in Fig. 2, step S1: a silicon-containing polycarbonate type polyurethane solution is provided. Step S2: a silicon-containing polycarbonate type polyurethane solution is sprayed to form a silicon-containing polycarbonate type polyurethane elastomer. Step S3: the silicon-containing polycarbonate type polyurethane elastomer is collected to form a first pipe element 11 composed of the silicon-containing polycarbonate type polyurethane elastomer. Step S4: a polycarbonate type polyurethane solution is provided. Step S5: the polycarbonate type polyurethane solution sprayed to wrap the outer surface of the first pipe element 11 to form a polycarbonate type polyurethane elastomer, in which the polycarbonate type polyurethane elastomer is the second pipe element 12. Step S6: a coil winding structure 20 is formed on the outer surface of the first pipe element 11 or the outer surface of the second pipe element 12. Further description of steps S1 to S6 is shown as followed.

First, a 10wt%∼20wt% silicon-containing polycarbonate type polyurethane solution is selected and placed inside an auto-sampler syringe. At an injection rate of 0.5 to 5.0 ml/hour, under a voltage of 12kV to 28kV and a sample collecting distance between 10 cm to 25 cm, the silicon-containing polycarbonate type polyurethane solution is sprayed through electrostatic spinning technology to produce silicon-containing polycarbonate type polyurethane fibers, of which the silicon-containing polycarbonate type polyurethane fibers are collected by a deposition electrode (not shown in the figure). In this embodiment, the deposition electrode is a rotation axis, and the layered silicon-containing polycarbonate type polyurethane elastomer obtained by the deposition electrode is the inner pipe of the polymer fiber tubular structure 1, the first pipe element 11. Immediately after, a polycarbonate type polyurethane solution of 10 wt% to 20 wt% was replaced in the auto-sampler syringe, and at the injection rate of 0.5 to 5.0 ml / hour, under a voltage of 12 kV to 28 kV and a sample collecting distance between 10cm to 25cm. The polycarbonate type polyurethane solution is sprayed through electrostatic spinning technology to produce polycarbonate type polyurethane fibers, and the polycarbonate type polyurethane fiber is collected on the outer surface of the first pipe element 11, covering the first pipe element 11, so the layered polycarbonate type polyurethane elastomer forms an outer pipe fitting, the second pipe element 12. In the embodiment of the present invention, the thickness of the first pipe element 11 accounts for 30% to 70% of the total thickness of the polymer fiber tubular structure 1, and the thickness of the second pipe element 12 accounts for 20% ∼ 80% of the total thickness of the polymer fiber tubular structure 1.

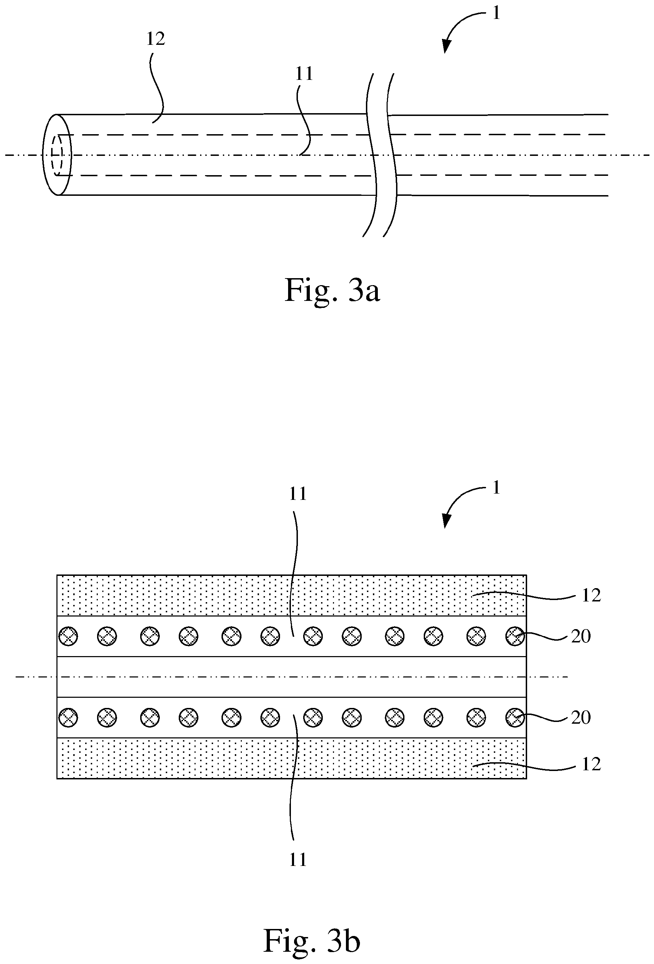

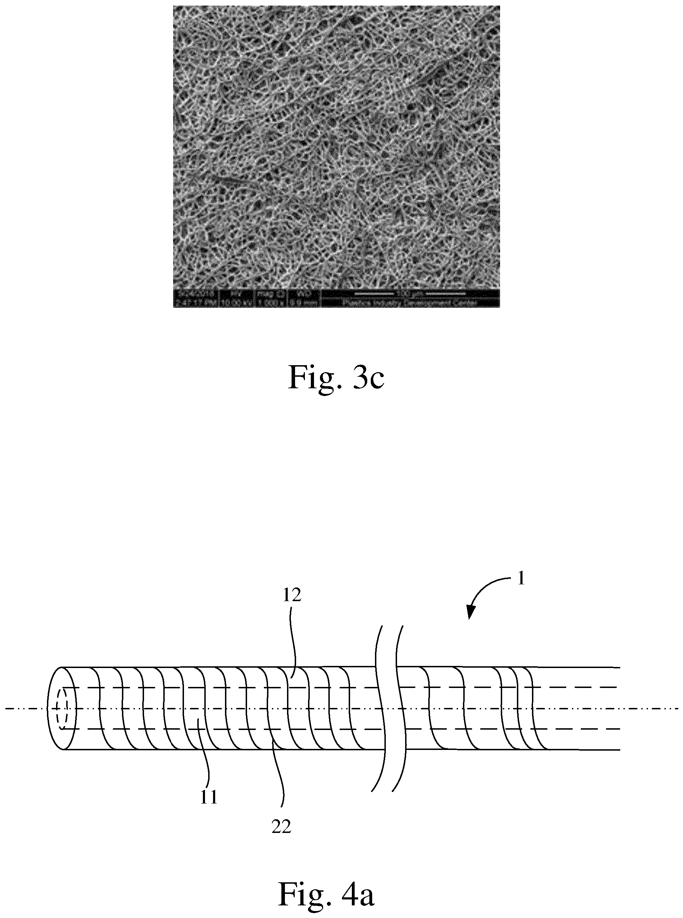

Please refer to Fig. 3a through Fig. 3c. Fig. 3a is a schematic view showing a coil winding structure 20 embedded into the outer surface of the first pipe element 11 in the polymer fiber tubular structure 1, Fig. 3b is a coil winding structure 20 embedded into the outer surface of the first pipe element 11 in the polymer fiber tubular structure 1. The cross-sectional schematic diagram and Fig. 3c shows a SEM image of the inner fiber layer of the first pipe of the polymer fiber tubular structure 1.

Please refer to Fig. 3a and Fig. 3b, the polymer fiber tubular structure 1 includes the first pipe element 11 and the second pipe element 12, and a coil winding structure 20 (the structure shown by the dashed line in Fig. 2a) is also included between the first pipe element 11 and the second pipe element 12. The coil winding structure 20 is embedded into the outer surface of the first pipe element 11, serving the purpose to prevent the polymer fiber tubular structure 1 to kink, therefore avoids blood flow obstruction problems.

In this embodiment, the coil winding structure 20 is formed on the outer surface of the first pipe element 11 after the first pipe element 11 is formed, then the coil winding structure 20 is wound onto the outer surface of the first pipe element 11, and then followed by wrapping the second pipe element 12 surround the first pipe element 11 and the coil winding structure 20. In another embodiment of the present invention, during the formation process of the first pipe element 11 and the second pipe element 12, the coil winding structure 20 wounds in the space between the first pipe element 11 and the second pipe element 12, which is, the contact position between the outer surface of the first pipe element 11 and the inner surface of the second pipe element 12. It should be noted, as shown by L1 in Fig. 2a, that the spacing distance of the coil winding structure 20 embedded into the outer surface of the first pipe element 11 may be the same, the spacing distance ranges from 100 µm to 1000 µm. In another preferred embodiment, the spacing distance of the coil winding structure 20 embedded into the outer surface of the first pipe element 11 represented as symbol L2, L3 or L4. That is, the spacing distance of the coil winding structure 20 embedded into the outer surface of the first pipe element 11 can be set according to demands. Thus, the spacing distance of the coil winding structure 20 embedded into the outer surface of the first pipe element 11 can be an equal spacing distance as shown in L1, or as the spacing distance indicated in L2, L3 and/or L4, and the coil winding structure 20 also can be wound around the outer surface of the first pipe element 11 at the same time. Therefore, according to the abovementioned, by performing SEM scanning on the polymer fiber tubular structure 1 as shown in Fig. 3a and Fig. 3b, can obtain the SEM image of the inner layer (as of the interface of outer surface of the first pipe element 11 and the inner surfaces of the second pipe element 12) of the polymer fiber tubular structure 1 as shown in Fig. 3c, in addition, it can also be confirmed from Fig. 3c that the inner layer of the polymer fiber tubular structure 1 has a specific fiber diameter and holes.

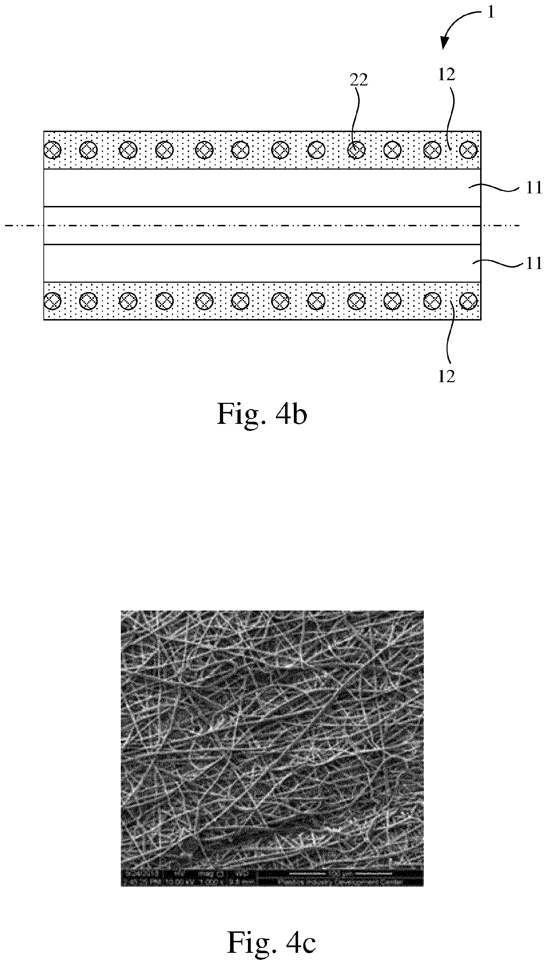

Next, please refer to Fig. 4a through Fig. 4c. Fig. 4a is a schematic view showing a coil winding structure 20 embedded into the outer surface of a first pipe element 11 in a polymer fiber tubular structure 1, and Fig. 4b is a schematic cross-sectional view of a coil winding structure 22 embedded into the outer surface of the second pipe element 12 in the polymer fiber tubular structure 1, and Fig. 4c is a SEM image showing the fiber structure of the outer layer of the second pipe element 12 in the polymer fiber tubular structure 1.

In Fig. 4a and Fig. 4b, after the second pipe element 12 is formed, the coil winding structure 22 is wound on the outer surface of the second pipe element 12. Similarly, the coil winding structure 22 is embedded into the second pipe element 12, with a spacing distance in the range of 100 µm to 1000 µm with the outer surface, and the spacing distance of the coil winding structure 22 may be the same or different. In addition, in the embodiment of the present invention, the diameter of the coil winding structure 20 and the coil winding structure 22 ranges from 100 µm to 500 µm. Similarly, when scanning the polymer fiber tubular structure 1 as shown in Fig. 4a and Fig. 4b, the SEM drawing of the outer layer (as the outer surface of the second pipe element 12) as shown in Fig. 3c. Therefore, the outer layer of the polymer fiber tubular structure 1 has a specific fiber diameter and holes.

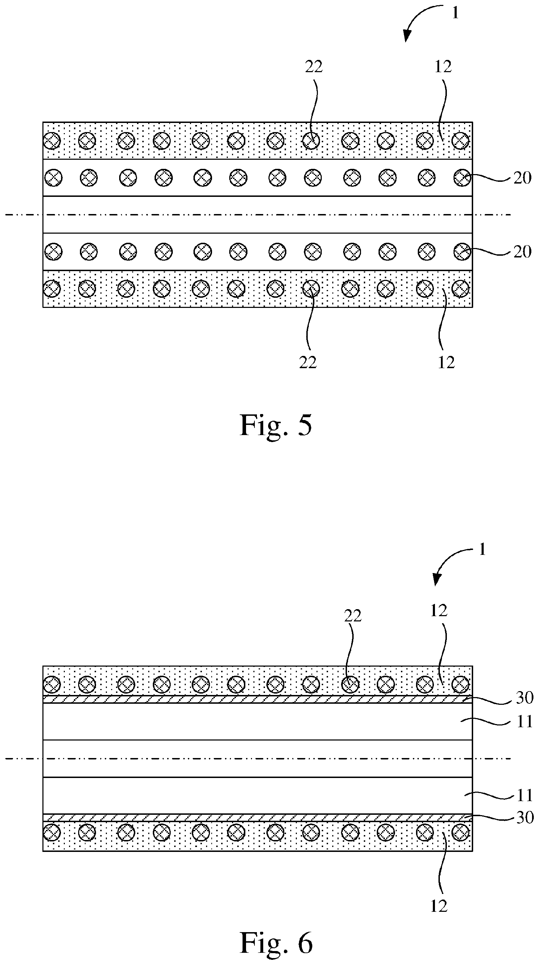

Another embodiment of the present invention is shown in Fig. 5. Fig. 5 is a schematic cross-sectional view showing the coil winding structure 20 embedded in the inner surface of the first pipe element 11 and the outer surface of the second pipe element 12 in the polymer fiber tubular structure 1. Fig. 5 which is to combine aforementioned Fig. 3a to Fig. 3b and Fig. 4a to Fig. 4b, that is, the coil winding structure 20 is wrapped around the surface of the first pipe element 11 after the first pipe element 11 is formed; or the coil winding structure 20 can be wound between the first pipe element 11 and the second pipe element 12, during the process of forming the first pipe element 11 and the second pipe element 12. Next, after the second pipe element 12 is formed, the coil winding structure 22 is wound on the outer surface of the second pipe element 12. In this embodiment, the spacing distance between the coil winding structure 20 on the outer surface of the first pipe element 11 and the coil winding structures 22 on the outer surface of the second pipe element 12 may be the same or different.

In addition, in another embodiment of the present invention, the polymer fiber tubular structure 1 further comprises an adhesive layer 30 disposed between the first pipe element 11 and the second pipe element 12, as shown in Fig. 6. In order to make an explanation, Fig. 6 is an example of the structure in Fig. 3b, but the adhesive layer 30 may be disposed between the first pipe 11 and the second pipe element 12 as shown in Fig. 3b or Fig. 5. In Fig. 6, the preparation method includes: first, coat an adhesive layer 30 in the middle of the first pipe element 11 or the second pipe element 12, then wind the coil winding structure 22 on the adhesive layer 30 or embedded it into the adhesive layer 30, and then, the second pipe element 12 is formed to cover the adhesive layer 30, the coil winding structure 22 and the first pipe element 11. In one embodiment, the adhesive layer 30 may be inserted between the outer surface of the first pipe element 11 and the coil winding structure 22 in an alternating manner. In the embodiment of the present invention, the adhesive layer 30 is dense polyurethane.

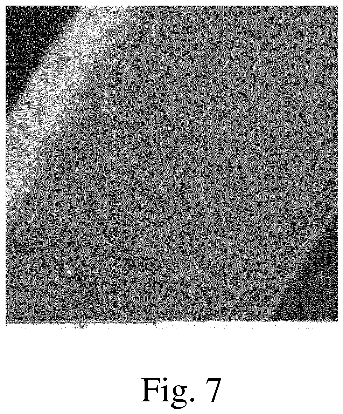

According to the above, as disclosed in the present invention the results from examine the polymer fiber tubular structure 1 by performing scanning electron microscope/energy dispersive X-ray mapping (SEM/EDS mapping) is shown in Fig.7, the red dots represents silicon. It can also prove that the first pipe element 11 is composed of silicon-containing polycarbonate type polyurethane, and the second pipe element 12 is composed of non-silicon-containing polycarbonate type polyurethane.

## Claims

1. A polymer fiber tubular structure (1), **characterized in that**:
a first pipe element (11), which is composed of a silicon-containing polycarbonate type polyurethane elastomer, including an inner surface and an outer surface;
a second pipe element (12), which is composed of a polycarbonate type polyurethane elastomer, including an inner surface and an outer surface, the second pipe element (12) is wrapped on the outer surface of the first pipe element (11), such that the first pipe element (11) and the second pipe element (12) are concentric structures; and
a coil winding structure (20, 22), embedded into the outer surface of the first pipe element (11) or the outer surface of the second pipe element (12).

2. The polymer fiber tubular structure (1) according to claim 1, wherein a thickness of the first pipe element (11) accounts for a total thickness ranging from 30% ∼ 70% of the polymer fiber tubular structure (1), and a thickness of the second pipe element (12) accounts for the total thickness ranging from 20% ∼ 80% of the polymer fiber tubular structure (1).

3. The polymer fiber tubular structure (1) according to claim 1, wherein the first pipe element (11) and the second pipe element (12) is a polymer fiber structure.

4. The polymer fiber tubular structure (1) according to claim 1, wherein the coil winding structure (20) is comprised of polyethylene terephthalate (PET).

5. The polymer fiber tubular structure (1) according to claim 4, wherein the diameter of the coil winding structure (20) ranges from 100 µm to 500 µm.

6. The polymer fiber tubular structure (1) according to claim 1, wherein the coil winding structure (20) comprised a spacing distance, the range of the spacing distance can be the same or different on the outer surface of the first pipe and the outer surface of the second pipe.

7. The polymer fiber tubular structure (1) according to claim 5, wherein the spacing distance ranges from 100 µm to 1000 µm.

8. The polymer fiber tubular structure (1) according to claim 1, wherein an adhesive layer (30) is disposed between the first pipe element (11) and the second pipe element (12).

9. The polymer fiber tubular structure (1) according to claim 1, wherein the adhesive layer (30) is alternately inserted between the outer surface of the first pipe element (11) and the coil winding structure.

10. The polymer fiber tubular structure (1) according to claim 9, wherein the adhesive layer (30) is polyurethane.

11. A method for preparing a polymer fiber tubular structure (1), the steps comprise:
forming a first pipe element (11), the first pipe element (11) includes an inner surface and an outer surface, the steps of forming the first pipe comprise:
providing a silicon-containing polycarbonate type polyurethane solution;
spraying the silicon-containing polycarbonate type polyurethane solution to form a silicon-containing polycarbonate type polyurethane fiber; and
collecting the silicon-containing polycarbonate type polyurethane fiber to obtain a silicon-containing polycarbonate type polyurethane elastomer to composed the first pipe element (11);
forming a second pipe element (12) to wrap the outer surface of the first pipe element (11), such that the first pipe element (11) and the second pipe element (12) are concentric structures, and the second pipe element (12) includes an inner surface and an outer surface, the steps of forming the second pipe comprise:
providing a polycarbonate type polyurethane solution; and
spraying the polycarbonate type polyurethane solution to wrap the outer surface of the first pipe to form a polycarbonate type polyurethane elastomer, wherein the polycarbonate type polyurethane elastomer is the second pipe element (12); and
forming a coil winding structure (20, 22) on the outer surface of the first pipe element (11) or on the outer surface of the second pipe element (12).

12. The method for preparing a polymer fiber tubular structure (1) according to claim 11, wherein a thickness of the first pipe element (11) accounts for a total thickness ranging from 30% ∼ 70% of the polymer fiber tubular structure (1), and a thickness of the second pipe element (12) accounts for the total thickness ranging from 20% ∼ 80% of the polymer fiber tubular structure (1).

13. The method for preparing a polymer fiber tubular structure (1) according to claim 11, wherein a concentration range of the silicon-containing polycarbonate type polyurethane solution and a concentration range of the polycarbonate type polyurethane solution are 10wt% ∼ 20wt%.

14. The method for preparing a polymer fiber tubular structure (1) according to claim 11, wherein a spacing distance of the coil winding structure (20, 22) on the outer surface of the first pipe element (11) or the outer surface of the second pipe element (12) may be the same or different.

15. The method for preparing a polymer fiber tubular structure (1) according to claim 11, wherein the spacing distance ranges from 100 µm to 1000 µm.
